# EUROPEAN PATENT SPECIFICATION

(11) **EP 2 220 959 B1**
(45) Date of publication and mention of the grant of the patent: **18.04.2018**
(21) Application number: 09180209.0
(22) Date of filing: 21.12.2009
(51) Int. Cl.: A45D 33/00, A61K 8/00, A61Q 1/00, B30B 11/00, B65B 1/24, A61Q 1/12, A61K 8/02

(54) **Decorated baked cosmetic product**
Verziertes gehärtetes Kosmetikprodukt
Produit cosmétique cuit décoré

(30) Priority: 23.12.2008 IT MI20082317
(43) Date of publication of application: 25.08.2010
(73) Proprietor: CHROMAVIS S.p.A., 20122 Milan (IT)
(72) Inventor: Priore, Romualdo, 20129 Milano (IT); Bigatto, Guido, 26010 Vaiano Cremasco (CR) (IT)
(74) Representative: Ripamonti, Enrico

(56) References cited:
- EP-A- 0 328 793
- EP-A- 0 432 808
- EP-A- 0 486 394
- EP-A- 1 837 278
- WO-A-2004/103690
- US-A- 5 861 165
- US-A- 6 058 942

## Description

The present invention relates to the process for decorating a baked cosmetic product.

Known baked cosmetic products are presented to the public in the form of spherical caps of product resting on a support generally formed of terracotta. In this respect, being transpirable, the terracotta facilitates evaporation of the solvent (generally water) therethrough and perfect drying of the product core.

After being dried, known "baked" products are cleaned both on their surface to eliminate any physiological crust which is formed by evaporation and reduces withdrawal, and laterally (along the disc circumference) to remove any product excess.

Consequently the aesthetic characteristics of the finished products remain simply those conferred by the structure of the cosmetic product itself. For example a marble effect can be obtained (by simply dispensing and mixing several colours) or a double or multi-stripe effect (by depositing tongues of different colours on the disc) or a patchwork effect (by depositing spots of different colours on the disc).

To render the product more attractive to the final consumer it is usual to create writing (personalization) or decorations on the product surface. These decorations are achieved for example by removing material, for example by laser incision or by milling the product surface only after baking.

Consequently the known technique does not enable well visible and attractive decorations to be created on the surface of this type of cosmetic product.

EP1837278 **discloses a process comprising**
- **a first step of introducing cosmetic powder in at least one container (the container is a metal container (column 3, lines 29-30)),**
- **compressing said powder until it forms at least one compacted agglomerate,**
- **forming at least one cavity within said agglomerate or between a plurality of reciprocally adjacent compacted agglomerates,**
- **adding further powders until said cavity is filled,**
- **slightly compressing said further powders,**
- **removing a surface layer of said further powders, and**
- **finally compressing all of the powders.**

An object of the present invention is therefore to provide a cosmetic product and a process for its formation which are improved compared with the known art.

A further object of the present invention is to provide a cosmetic and a process able to create a well defined non-random aesthetic effect on the product, hence considerably improving its visual attractiveness.

These and other objects are attained by providing a cosmetic product and a process for its formation in accordance with the technical teachings of the accompanying claims.

Further characteristics and advantages of the invention will be apparent from the description of a preferred but non-exclusive embodiment of the invention, illustrated by way of non-limiting example in the accompanying drawings, in which:
Figures 1-5 schematically represent steps in the production of the cosmetic product according to the present invention;
Figure 6 shows a section through the cosmetic product of the present invention;
Figure 7 is an enlarged detailed view of a part of Figure 6;

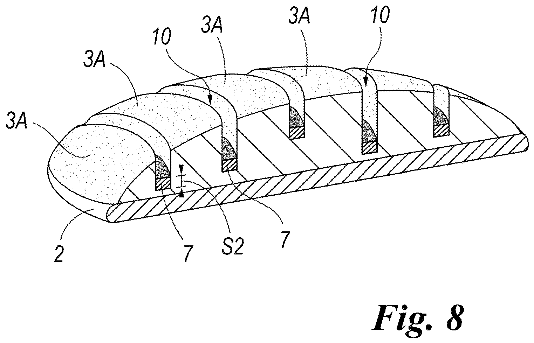

Figure 8 is a partially sectional perspective view of the product in question.

Said figures show a cosmetic indicated overall by the reference numeral 1.

The procedure for forming the cosmetic 1 is as follows.

A disc of permeable material is firstly provided. The term "permeable material" means a material which enables transpiration of water vapour (or other solvent), for reasons which will be apparent hereinafter. The disc 2 is advantageously of terracotta.

The cosmetic paste presents a high percentage of volatile component, between 20 and 60% by weight (preferably 40%), which enables the compound to be dispensed as a creamy paste.

The cosmetic paste is then pre-formed by a preferably smooth first die 4, which can for example have a concave shape as in the accompanying figures, or a flat shape. Advantageously before closing the die (arrow F), a cloth 5, preferably of microporous fibre, is interposed between the paste 3 and the die.

After opening the die 4 (arrow G), the paste is covered completely with a layer of a powdery cosmetic material. The powdery cosmetic material in question is particularly a cosmetic powder of any type, preferably pearlescent powders, pigments, or mixtures.

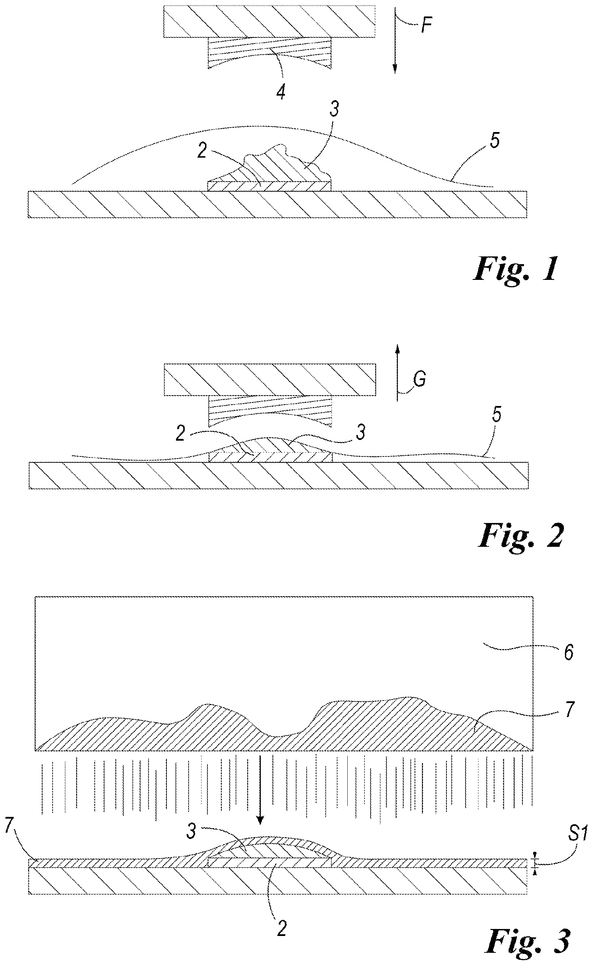

The entire surface is covered (Figure 3) using a sieve 6. The powder which falls onto the paste 3 remains on it by sticking to it, given that this is still wet. Essentially a layer is formed on the paste having a height S1 which, depending on the result to be obtained, can vary from 0.2 to 1 mm, preferably 0.6 mm.

The paste 3 covered with cosmetic powder 7 is then compacted by means of a second die 8 presenting on its surface at least one relief 8A able to form on the product surface at least one recess 10, on the base of which the powder 7 hence remains pressed.

The reliefs 8A are disposed on the die such as to represent a decoration. In the example they are disposed such as to form parallel lines which extend in a direction perpendicular to the sheet carrying the drawing. Each line has a different depth. In alternative embodiments, the relief or reliefs can represent a shape of flowers, letters, images or any decorative element. Advantageously the reliefs have a maximum height which is related to the height of the paste 3. In particular the reliefs have a height between 5% and 40% of the height of the paste 3.

Advantageously a further cloth (not shown) can be interposed during the pressing step with the second die.

After opening the die (arrow L) the cosmetic product is baked to facilitate loss of the solvent component. Advantageously, given the permeability of the disc 2, the solvent (preferably water) is quickly released even from the core of the cosmetic product by passing through the disc 2 which, by virtue of its permeability, does not prevent the evaporated liquid component from abandoning the product.

Baking takes place for example in an atmospheric pressure oven at a temperature between 30ºC and 85ºC, preferably 60ºC, for a time of 12 h, to achieve a residual solvent percentage by weight indicatively less than 1%. The cosmetic paste 3 is hence dry on termination of baking.

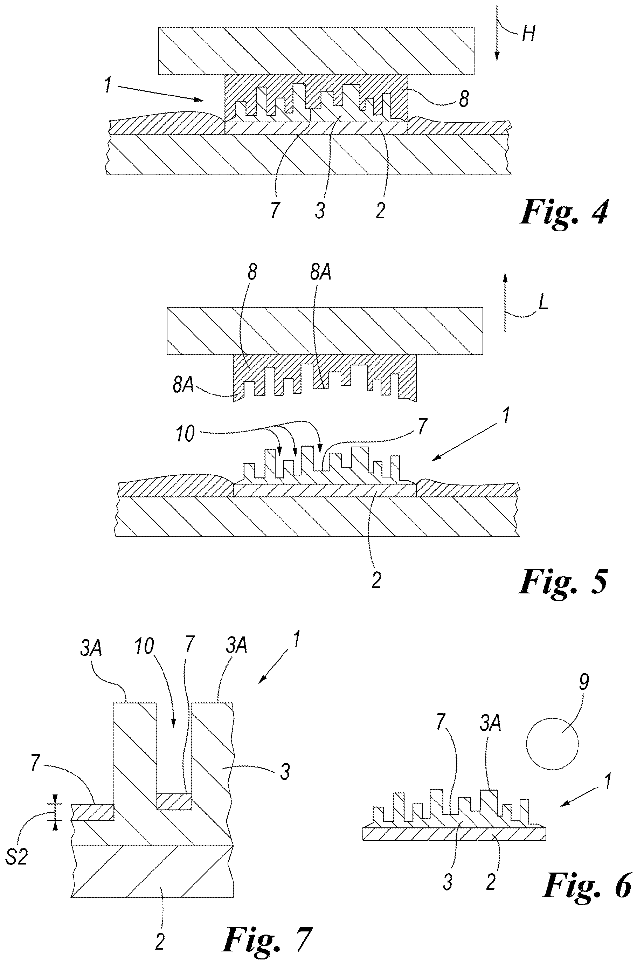

After baking, the cosmetic is surface-cleaned by rubbing, for example on an abrasive sponge 9 which completely removes that powdery material (now integral with the paste) which remains on the external surface, to hence leave this powdery product only within those recesses not reached by the rubbing. As can be seen from the enlargement of Figure 7, the baked paste 3 remains visible on the surface 3A rubbed by the sponge 9, while on the base of the recesses 10 a layer 7 of powdery product remains visible having a height S2 of between 0.1 and 0.5 mm.

This rubbing hence gives the cosmetic product an engaging visual appearance given by the precision of the decoration on the surface. In particular the predominant colour is that of the paste forming most of the product, the decoration itself involving only the base of the recesses formed on the surface.

A preferred embodiment of the invention has been described, however others can be conceived using the same inventive concept. Hence the products used for the cosmetic paste 3 can be of any known type, the powdery product 7 used being any cosmetic product suitable for the purpose.

## Claims

1. A process for forming a decorated cosmetic, comprising the steps of:
a. depositing a cosmetic paste comprising a solvent component on a disc of permeable material;
b. pre-forming the cosmetic paste with a die;
c. covering the cosmetic paste with a powdery cosmetic material to form a layer thereon;
d. compacting the cosmetic paste and the powdery material with a second die presenting at least one relief in such a manner as to form at least one recess in the surface of the cosmetic paste;
e. baking the cosmetic paste to facilitate loss of the solvent component;
f. cleaning the surface of the cosmetic to remove the powdery material from the surface, and hence leave it only within said recesses.

2. A process as claimed in the preceding claim, **characterised in that** before pre-forming the paste, a cloth is rested on it to remain interposed between the paste and the first die during pre-forming.

3. A process as claimed in one or more of the preceding claims, **characterised in that** the cloth is of porous microfibre.

4. A process as claimed in one or more of the preceding claims, **characterised in that** the covering is achieved by sprinkling the powdery material over the paste by means of a sieve.

5. A process as claimed in one or more of the preceding claims, **characterised in that** said deposited layer has a thickness between 0.2 and 1 mm.

6. A process as claimed in one or more of the preceding claims, **characterised in that** a further cloth is interposed during compacting with the second die.

7. A process as claimed in one or more of the preceding claims, **characterised in that** before being dried, said paste comprises a percentage of water by weight between 20 and 60%, preferably 40%.

8. A decorated cosmetic product comprising a disc formed of permeable material, on which a dried cosmetic paste is disposed in its surface, said cosmetic paste presenting recesses, on the base of which a layer of cosmetic powder is present laying on a layer of cosmetic paste, **characterised in that** said cosmetic powder layer is present only within said recesses, and **characterised in that** said cosmetic powder present on the base of the recesses is compacted.

9. A cosmetic product as claimed in claim 8, **characterised in that** the layer present on the base of the recesses has a height between 0.1 and 0.5 mm.

10. A cosmetic product or process as claimed in one or more of the preceding claims, **characterised in that** the powdery material is chosen from one or more of the following elements: pearlescent pigments, pigments, pearlescent elements.

11. A cosmetic product or process as claimed in one or more of the preceding claims, **characterised in that** said permeable material is terracotta.

## Patentansprüche

1. Verfahren zum Bilden eines verzierten Kosmetikums, umfassend die folgenden Schritte:
a. Aufbringen einer Kosmetikpaste umfassend eine Lösemittelkomponente auf einer Scheibe aus durchlässigem Material;
b. Vorformen der Kosmetikpaste durch eine Matrize;
c. Beschichten der Kosmetikpaste mit einem kosmetischen pulverförmigen Material zum Bilden einer Schicht darauf;
d. Kompaktieren der Kosmetikpaste und das pulverförmige Material durch eine zweite Matrize, die mindestens einen Vorsprung derart aufweist, dass mindestens eine Ausnehmung in der Oberfläche der Kosmetikpaste gebildet wird;
e. Erhitzen der Kosmetikpaste, um den Verlust der Lösemittelkomponente zu begünstigen;
f. Reinigen der Oberfläche des Kosmetikums, um das pulverförmige Material aus der Oberflache zu entfernen und deshalb dasselbe nur innerhalb der genannten Ausnehmungen zu halten.

2. Verfahren nach dem vorherigen Anspruch, **dadurch gekennzeichnet, dass** vor dem Vorformung der Paste ein Tuch darauf angelegt wird, das zwischen der Paste und der ersten Matrize bei der Vorformung eingesetzt bleibt.

3. Verfahren nach einem oder mehreren der vorangegangenen Ansprüche, **dadurch gekennzeichnet, dass** das Tuch aus poröser Mikrofaser besteht.

4. Verfahren nach einem oder mehreren der vorangegangenen Ansprüche, **dadurch gekennzeichnet, dass** die Beschichtung durch Bestreuen der Paste mit dem pulverförmigen Material durch ein Sieb erfolgt.

5. Verfahren nach einem oder mehreren der vorangegangenen Ansprüche, **dadurch gekennzeichnet, dass** die genannte aufgebrachte Schicht eine Dicke von 0,2 bis 1 mm hat.

6. Verfahren nach einem oder mehreren der vorangegangenen Ansprüche, **dadurch gekennzeichnet, dass** ein weiteres Tuch beim Kompaktieren mit der zweiten Matrize eingesetzt wird.

7. Verfahren nach einem oder mehreren der vorangegangenen Ansprüche, **dadurch gekennzeichnet, dass** die genannte Paste vor dem Trocknen ein Gewichtsanteil an Wasser von 20 bis 60%, vorzugsweise 40%, umfasst.

8. Verziertes Kosmetikprodukt umfassend eine aus durchlässigem Material bestehende Scheibe, wobei eine getrocknete Kosmetikpaste auf ihrer Oberfläche angeordnet ist, wobei die genannte Kosmetikpaste Ausnehmungen aufweist, wobei an der Base davon eine Schicht von Kosmetikpulver vorhanden ist und auf einer Schicht von Kosmetikpaste liegt, **dadurch gekennzeichnet, dass** die genannte Schicht von Kosmetikpulver nur innerhalb der genannten Ausnehmungen vorhanden ist, und **dadurch gekennzeichnet, dass** das genannte, auf der Base der Ausnehmungen vorhandene Kosmetikpulver kompaktiert wird.

9. Kosmetikprodukt nach Anspruch 8, **dadurch gekennzeichnet, dass** die auf der Base der Ausnehmungen vorhandene Schicht eine Höhe von 0,1 bis 0,5 mm hat.

10. Kosmetikprodukt oder Verfahren nach einem oder mehreren der vorangegangenen Ansprüche, **dadurch gekennzeichnet, dass** das pulverförmige Material aus einem oder mehreren der folgenden Elementen gewählt wird: Perlglanzpigmente, Pigmente, Perlglanzelemente.

11. Kosmetikprodukt oder Verfahren nach einem oder mehreren der vorangegangenen Ansprüche, **dadurch gekennzeichnet, dass** das genannte durchlässige Material Terrakotta ist.

## Revendications

1. Procédé pour former un cosmétique décoré, comprenant les étapes de :
a. déposer une pâte cosmétique comprenant un composant solvant sur un disque de matériau perméable ;
b. préformer la pâte cosmétique avec une matrice ;
c. couvrir la pâte cosmétique par un matériau cosmétique pulvérulent pour former une couche sur elle ;
d. compacter la pâte cosmétique et le matériau pulvérulent avec une seconde matrice présentant au moins un relief de sorte à former au moins un renfoncement dans la surface de la pâte cosmétique ;
e. cuire la pâte cosmétique afin de favoriser la perte du composant solvant ;
f. nettoyer la surface du cosmétique pour éliminer le matériau pulvérulent de la surface, le laissant ainsi seulement dans lesdits renfoncements.

2. Procédé selon la revendication précédente, caractérisé en que, avant de préformer la pâte, un drap est posé sur elle de sorte à demeurer interposé entre la pâte et la première matrice pendant le préformage.

3. Procédé selon l'une ou plusieurs des revendications précédentes, **caractérisé en ce que** le drap est en microfibre poreuse.

4. Procédé selon l'une ou plusieurs des revendications précédentes, **caractérisé en ce que** la couverture est obtenue en déposant du matériau pulvérulent sur la pâte moyennant un crible.

5. Procédé selon l'une ou plusieurs des revendications précédentes, **caractérisé en ce que** ladite couche déposée a une épaisseur entre 0,2 et 1 mm.

6. Procédé selon l'une ou plusieurs des revendications précédentes, **caractérisé en ce que** un drap supplémentaire est interposé pendant le compactage avec la seconde matrice.

7. Procédé selon l'une ou plusieurs des revendications précédentes, **caractérisé en ce que**, avant le séchage, ladite pâte comprend un pourcentage en poids d'eau entre 20 et 60%, préférablement 40%.

8. Produit cosmétique décoré comprenant un disque de matériau perméable, une pâte cosmétique séchée étant agencée sur sa surface, ladite pâte cosmétique présentant des renfoncements, sur la base desquels une couche de poudre cosmétique est présente, étendue sur une couche de pâte cosmétique, **caractérisé en ce que** ladite couche de poudre cosmétique est présente seulement à l'intérieur desdits renfoncements, et **caractérisé en ce que** ladite poudre cosmétique présente sur la base des renfoncements est compactée.

9. Produit cosmétique selon la revendication 8, **caractérisé en ce que** la couche présente sur la base des renfoncements a une hauteur entre 0,1 et 0,5 mm.

10. Produit cosmétique ou procédé selon l'une ou plusieurs des revendications précédentes, **caractérisé en ce que** le matériau pulvérulent est choisi entre l'un ou plusieurs des éléments suivants : pigments nacrés, pigments, éléments nacrés.

11. Produit cosmétique ou procédé selon l'une ou plusieurs des revendications précédentes, **caractérisé en ce que** ledit matériau perméable est terre cuite.
